# EUROPÄISCHE PATENTANMELDUNG

(11) **EP 0 948 948 A2**
(43) Veröffentlichungstag der Anmeldung: **13.10.1999**
(21) Anmeldenummer: 99105459.4
(22) Anmeldetag: 17.03.1999
(51) Int. Cl.: A61F 6/18

(54) **Einführungsgerät zum Einbringen einer intrauterinen empfängnisverhütenden Vorrichtung**

(30) Priorität: 07.04.1998 DE 19815552
(71) Anmelder: Riek, Siegfried, Dr. med., D-78628 Rottweil (DE)
(72) Erfinder: Riek, Siegfried, Dr. med., D-78628 Rottweil (DE)

(57) **Zusammenfassung**

Einführungsgerät 1 zum Einlegen einer intrauterinen empfängnisverhütender Vorrichtung (IUP) 11 in die Gebärmutterhöhle. Das Einführungsgerät 1 besteht aus einem Hohlstab 2, der am distalen Ende zwei nach distal offene Längsschlitze 3 und 3' besitzt. Auf dem Hohlstab 2 ist ein Schieber 6 angebracht und dient zur Einstellung der Sondenlänge. Weiterhin ist ein Halteelement 7 auf dem Hohlstab angebracht. Der Hohlstab besitzt Positionierungselemente 4 und 4' , welche das IUP im Hohlstab in definierter Position halten. Ein rohrförmiger Stempel 8 ist im Hohlstab leicht verschiebbar und besitzt einen Vorschiebegriff 9 welcher verschiebbar auf dem Stempel angebracht ist. Die empfängnisverhütende Vorrichtung besteht aus einem Spiralenkörper 14, 2 elastischen Armen 12 und 12' und einem Befestigungsteil 15 am proximalen Ende. Das Einführungsgerät erleichtert das Einlegen des IUP in die Gebärmutterhöhle und ist einhändig zu bedienen. Verletzungen werden verhindert und eine korrekte anatomische Lage des IUP in der Gebärmutterhöhle wird gewährleistet.

## Beschreibung

IUP sind T-förmige zylindrische Kunststoffkörper mit zwei elastischen Armen am cranialen Ende des Körpers und einer Kupferdrahtumwicklung oder Hormonbeschichtung am vertikalen Spiralenkörper, welche zur Einlage in die Gebärmutterhöhle bestimmt sind. Um die in der gynäkologischen Praxis routinemäßige Einlage zu bewerkstelligen, ist ein Einführungsgerät notwendig, um das IUP durch die Vagina und den Gebärmutterhals bis in die Gebärmutterhöhle vorschieben zu können und in anatomisch korrekter Lage zu positionieren. Durch die T-Form hat das eingelegte IUP Halt in der dreieckigen Gebärmutterhöhle, wobei die entfalteten horizontalen Arme in den Tubenecken der Gebärmutterhöhle zu liegen kommen und der vertikale Spiralenkörper zum Gebärmutterhals zeigt. Die Gebärmutterhöhle ist ein dreidimensionaler Raum, welcher von Gebärmutterschleimhaut ausgekleidet ist und die Form eines dreieckigen, an der Vorder- und Hinterwand abgeplatteten Kissens hat, wobei die horizontale Basisfläche cranial liegt und die Spitzen nach lateral zu den Tubenabgängen sowie caudal zum Gebärmutterhals zeigen. Die korrekte Lage des IUP in der Gebärmutterhöhle ist Voraussetzung für die komplikationslose Einlage, für die Beschwerdefreiheit der Patientin sowie für eine zuverlässige empfängnisverhütende Wirkung.

Gemäß dem Stand der Technik sind verschiedene Typen von IUP und verschiedene Einführungsgeräte bekannt (G 92 16 851.5, DE 43 18 941 A1, DE 42 41 662 A1, DE 42 37 950 C1, EP 0 584 628 A1, G 85 15 092.4). Alle diese genannten Vorrichtungen haben den Nachteil, daß eine klar definierte Positionierung des IUP mit einer Hand schlecht möglich ist, die Handhabung kompliziert ist, und zum Teil wie bei den zur Zeit von der Firma Schering (Berlin) angebotenen Einführungsgeräten für die IUP Mirena (gesch. Warenzeichen) und Nova-T (gesch. Warenzeichen) zweihändig manipuliert werden muß. So liegt das Problem darin, daß bei diesen Konstruktionen 3 Instrumente (Stempel, Einführungsrohr und Faßzange) mit 2 Händen gehalten und so manipuliert werden müssen, daß ihre Position zueinander und zur Faßzange verändert wird. Die Position der Faßzange muß jedoch wahrend des Einlegevorganges unverändert bleiben. Weiterhin ist die industrielle Herstellung von technisch komplexen Einführungsgeräten nicht sinnvoll, da diese nach der Einführung des IUP entsorgt werden müssen und daher eine wenig umweltbelastende und möglichst kostengünstige Produktion und Entsorgung anzustreben ist.

Der Stand der Technik ist in DE 42 37 950 C1 ausführlich dargelegt. Um die dem Stand der Technik zugrundeliegenden Probleme zu lösen wird in dieser Druckschrift ein Einführungsgerät mit einem am distalen Ende geschlossenen Rohr und proximal davon liegender seitlicher länglicher Öffnung beschrieben. Die seitliche Öffnung ist so breit, daß von der Rohrwand nur ein Steg stehenbleibt, der zur zentralen Längsachse des Einführungsgerätes hin verläuft und dort mit kugeliger Spitze endet und somit als distales Ende eines geschlossenen Rohres dargestellt ist. Über einen Stempel wird das IUP soweit vorgeschoben, bis die Arme des IUP aus dem Längsschlitz herausfedern. Danach muß das distale Ende an dem IUP vorbei zum Stempel hin nach proximal geschoben werden bis das IUP freigegeben ist. Dabei muß der Stempel seine Lage zur Gebärmutter beibehalten während das Einführungsgerät verschoben wird. Insbesondere ist keine konstante taktile Rückmeldung über den Stempel zu erwarten, da dieser das IUP nach distal drücken muß, während das keilförmig verdickte Stegende daran vorbeigezogen wird, zumal das IUP vom Stegende aus der zentralen Längsachse des Einführungsrohres herausgedrückt wird. Dabei entstehen veränderliche Gleitwiderstände , da der Steg , der konstruktionsgemäß das IUP im Einführungsgerät zurückhalten will , seine Lage zum IUP bis zum distalen Ende dauernd ändert. Daraus ergibt sich ein Risiko, da das IUP aus der Längsachse des Einführungsgerätes gedrückt wird und dabei die Gebärmutterwand verletzen kann, oder falls das Einführungsgerät nicht vollständig gegen den Stempel zurückgeschoben wird , das IUP sich am Stegende verhaken kann und dann das IUP unkontrolliert beim Entfernen des Einführungsgerätes nach distal gezogen wird und somit wiederum die Wände der Gebärmutter verletzt werden können. So ist es bei diesem Einführungsgerät nicht möglich das entfaltete IUP mit dem Stempel mit leichter Hand gegen die craniale Gebärmutterwand zu drücken und einen federnden Widerstand zu spüren. Verschiebt sich der Stempel beim Zurückschieben des Einführungsgerätes nur minimal um wenige Millimeter nach caudal, liegt das IUP nicht mehr korrekt. Da kein Fixpunkt zur Orientierung für die Lage des Stempels vorhanden ist, wird dies nur schwerlich gelingen.

Weiterhin ist der Stempel so gebaut, daß die Fäden des IUP zwischen dem hohlen Einführungsgerät und dem Stempel verlaufen. Somit ist es notwendig am Ende des Einführungsvorganges zunächst den Stempel aus dem Einführungsgerät zu entfernen und dann erst das Einführungsgerät zurückzuziehen, da sonst ein Verklemmen der Fäden zwischen Einführungsgerät und Stempel nicht ausgeschlossen werden kann. Würde dieses eintreten, könnte das IUP beim Entfernen des Einführungsgerätes mit eingesetztem Stempel wieder aus der Gebärmutterhöhle herausgezogen oder zumindest disloziert werden.

Um die gemäß dem Stand der Technik vorhandenen Probleme zu lösen, wird ein Hohlstab verwendet, welcher in einer vorteilhaften Ausführung zwei Öffnungen als Positionierungselemente besitzt, in die sich die Verdickungen der Arme des IUP teilweise einlegen können. Weiterhin werden vom distalen Ende des Hohlstabes her in dessen Wandung zwei Längsschlitze so angebracht, daß diese eine Entfaltung der Arme des IUP beim Vorschieben nach distal ermöglichen. Im Gegensatz zum beschriebenen Stand der Technik in der Druckschrift DE 42 37 950 C1 ist das distale Ende des Hohlstabes nicht geschlossen, sondern offen. Die Rohrwandanteile zwischen den Längsschlitzen können aus einem elastischen, federndem Material gebildet sein. Am distalen Ende der Rohrwandanteile sind Verdickungen so angebracht, daß diese den Innendurchmesser des Hohlstabes nicht verringern. Bei Druck von lateral können diese Rohrwandanteile sich jedoch zum Zentrum der zentralen Längsachse hin bis zur Berührung ihrer verdickten Enden bewegen, wobei diese eine kugelförmige Spitze des Einführungsgerätes bilden, welche im Durchmesser geringer als der Hohlstabdurchmesser ist und somit eine schonende und atraumatische Aufdehnung des Gebärmutterhalses gewährleistet ist. Wegen der Form der Enden des Hohlstabes ist eine Federbewegung nur hin zum Zentrum der Längsachse oder zurück in die Ausgangsposition möglich. In der Gebärmutterhöhle werden die Rohrwandanteile in ihre Ausgangsposition zurückkehren und den gesamten Innendurchmesser des Hohlstabes freigeben. Durch ihre verdickten Enden entsteht ein hoher Gewebewiderstand, so könnte die craniale Begrenzung der Gebärmutterhöhle wie mit einer Gebärmuttersonde ohne Risiko ertastet werden. Beim Vorschieben des IUP nach korrekter Positionierung des Einführungsgerätes können sich die Arme nun im oberen Drittel der Gebärmutterhöhle nach lateral entfalten, ohne die craniale Begrenzung zu erreichen. Dieses ist gemäß dem Stand der Technik auch mit dem in der Druckschrift DE 42 37 950 C1 beschriebenen Einführungsgerät möglich, jedoch wird gegenüber diesem Stand der Technik die Bewegung des IUP durch zwei nach distal offene Längsschlitze in der Rohrwand, welche exakt zur Längsachse des Hohlstabes verlaufen und mit Hilfe einer Markierung auf dem Hohlstab exakt nach lateral ausgerichtet werden können vorgeschrieben und die Arme zwischen den zwei Rohrwandanteilen so geschient, daß eine Drehbewegung des IUP, bei der die Arme dann nicht mehr nach lateral zeigen würden und eine Entfaltung dieser behindert wäre ausgeschlossen. Weiterhin kann durch diese Form des Hohlstabes das entfaltete IUP mit dem Stempel mit ganz geringem Druck soweit nach distal vorgeschoben werden bis die Arme die craniale Begrenzung der Gebärmutterhöhle erreichen. Durch eine entsprechend vorgegebene Position des Vorschiebegriffes auf dem Stempel kann auch bei starkem Druck gegen den Vorschiebegriff das IUP nicht weiter als bis zum Anschlag des Vorschiebegriffes am proximalen Ende des Hohlstabes vorgeschoben werden. Dadurch wird das Verletzungsrisiko in einer heiklen Phase des Einführungsvorganges deutlich reduziert. Wird das proximale Ende des Stempels mit dem Daumen nach distal gehalten während der Hohlstab mit Zeige- und Mittelfinger der gleichen Hand an einem Halteelement gegen den Daumen zurückgeschoben wird, ist durch eine sehr gute taktile Rückmeldung eine optimale feinmotorische Steuerung und Orientierung im Gegensatz zu zweihändigen Methoden vorhanden. Beim Zurückschieben des distalen Endes des Hohlstabes nach proximal spürt die Tastfäche des Daumens den federnden Widerstand des entfalteten IUP beim Andrücken gegen die kraniale Begrenzung der Gebärmutterhöhle. Dieser leichte Druck nach distal, welcher durch den federnden Widerstand konstant gehalten werden kann, meldet permanent die korrekte Lage des IUP, bis der Hohlstab und damit auch der Vorschiebegriff bis zu einer Markierung auf dem Stempel beziehungsweise bis zum proximalen Ende des Stempelrohres und somit bis zum Anschlag am Daumen zurückgeschoben ist und das IUP somit vollständig vom Hohlstab freigegeben ist. Nun kann das gesamte Einführungsgerät nach dem Wegnehmen des Daumens vom Stempel zwischen Zeige- und Mittelfinger zurückgezogen werden, da die Fäden des IUP im Gegensatz zum bekannten Stand der Technik in einem rohrförmigen Stempel verlaufen und damit kein Widerstand das Herausgleiten der Fäden aus dem Einführungsgerät behindern kann.

Gegenüber dem Stand der Technik wird bei der erfindungsgemäßen Konstruktion des Einführungsgerätes nach Einstellung der Sondenlänge ohne weitere Einstellmaßnahmen immer gewährleistet sein, daß die Arme des IUP sich im oberen Drittel der Gebärmutterhöhle exakt in der Längsachse des Einführungsgerätes exakt nach lateral im 90 Grad Winkel zur entsprechenden Markierung des Einführungsgerätes entfalten und die vollständig entfalteten Arme des IUP exakt an der cranialen Wand der Gebärmutterhöhle zu liegen kommen und nicht in eine Ebene, welche distal des Hohlstabes liegt geschoben werden können. Hieraus ergibt sich gegenüber dem Stand der Technik ein wesentlicher Sicherheitsvorteil für die Patientin Gegenüber dem Stand der Technik soll eine Kombination aus einem Einführungsgerät mit darin korrekt plaziertem IUP steril verpackt gefertigt werden. Der Arzt soll nur noch die Kombination aus der Verpackung nehmen, die Sondenlänge einstellen, und dann das IUP einhändig durch Betätigung des Vorschiebegriffes und des Haltegriffes einsetzen können. Damit ist auch eine nicht unwesentliche Zeitersparnis bei der Vorbereitung und beim Einlegen gewährleistet.

In der Druckschrift DE 4241662 A1 wird ein Einlegegerät beschrieben, welches in zerlegter Form an den Arzt geliefert wird und vom Arzt zunächst zusammengebaut werden muß. Dabei muß das IUP zunächst in das Einlegegerät eingesetzt werden. Dies ist ein zeitraubender und komplizierter Vorgang. Das Einführungsgerät besteht aus einem Hohlstab mit Längsschlitz, der zum proximalen Ende hin offen ist, einer Spange sowie einem Schieber und einem Gleitkörper, welcher den Schieber mit dem Stempel verbindet. Zur definierten Positionierung des IUP im Hohlstab besitzt dieser am distalen Ende zwei Aussparungen zur Aufnahme der Verdickungen der Arme des IUP. Diese Ausführung ist nicht besonders vorteilhaft, da die Produktion des Einführungsgerätes kompliziert ist, und das Zusammenbauen des Gerätes vor der Anwendung umständlich ist. Nach dem Zusammensetzen wird der Schieber auf die Sondenlänge eingestellt. Nach dem Einführen in die Gebärmutterhöhle kommt somit das nicht entfaltete IUP an der cranialen Begrenzung der Gebärmutterhöhle zu liegen. Wird nun der Hohlstab zurückgeschoben wollen sich die Arme zwar nach lateral entfalten, können jedoch durch den engen Kontakt zur cranialen Gebärmutterwand daran gehindert werden, so daß eine Entfaltung unterbleibt. Um die korrekte Plazierung des IUP zu erreichen müßte nach der Entfaltung der Arme das IUP soweit nach distal geschoben werden, bis die Arme der cranialen Gebärmutterwand anliegen. Somit ist mit diesem System eine korrekte Einlage nicht möglich. Um diese zu erreichen müßte der Stempel gegen den Widerstand des Schiebers nach distal geschoben werden, wobei jedoch der federnde Widerstand beim Erreichen der cranialen Wand der Gebärmutterhöhle nicht ertastet werden könnte, da der Gleitwiderstand , welcher beim Verschieben des Stempels gegen den Schieber auftritt dies unmöglich macht.

Weiterhin ist die Handhabung schwierig, weil der Stempel gehalten werden muß während der Hohlstab zurückgezogen wird. Beim unbeabsichtigten vordrücken des Stempels nach distal könnte die Gebärmutterwand verletzt werden, falls die Arme des IUP nicht vollständig entfaltet sind. Weiterhin muß der Stempel zunächst entfernt werden bevor der Hohlstab entfernt wird, da der Faden des IUP zwischen Stempel und Hohlstab verläuft und sich dort verklemmen kann. Sonst würde das IUP wieder herausgezogen werden oder disloziert werden.

In DE 43 18 941 A1 wird ein Einführungsgerät beschrieben, welches bedingt durch federnde Sollknickstellen am proximalen Ende des Hohlstabes sowie einer Abstützung dieser Federelemente am Stempel die Einlage eines IUP ermöglichen soll. Das Problem dabei ist, das auch hier ein technisch aufwendig herzustellendes Einführungsgerät beschrieben wird, das IUP vor der Einlage zunächst in das Einführungsgerät eingesetzt und korrekt plaziert werden muß. Weiterhin besteht der hohe Anspruch an die Federelemente sich beim Einführen des Hohlstabes durch den Gebärmutterhals nicht zu verschieben, jedoch dann, wenn das distale Ende des Einführungsgerätes die craniale Wand der Gebärmutterhöhle berührt. Entgegen der Beschreibung in der Druckschrift liegen die Werte dieser Drücke dicht beisammen, wobei bei engem Gebärmutterhals der Druck schon beim Durchschieben durch diesen recht hoch und über dem Berührungsdruck der Gebärmutterwand liegen kann. Liegt dieser Druck relativ hoch, kann er gleich wie der Perforationsdruck für die Wand der Gebärmutterhöhle sein. Somit kann dieses Gerät keine Orientierung bieten, da der dem Hohlstab entgegengesetzte Druck bei der Einlage variabel ist und zudem noch patientenspezifisch variiert. Somit ist dieses Gerät nicht geeignet die craniale Wand der Gebärmutterhöhle zu ertasten , zumal ein einstellbarer Schieber auch nicht vorhanden ist. Dieser Schieber könnte dann auf die gemessene Sondenlänge eingestellt werden und würde dem Untersucher die Orientierung geben, wo das distale Ende des Einführungsgerätes liegt. Weiterhin soll bei diesem Einführungsgerät die Spirale entfaltet werden, wenn Ihre Arme im unentfalteten Zustand noch der cranialen Wand der Gebärmutterhöhle anliegen, zumal dies die Entfaltung behindern kann. Die Lage des IUP ist somit nicht korrekt, da auch nach der Entfaltung eine Distanz zwischen den nach lateral entfalteten Armen und der cranialen Wand der Gebärmutterhöhle verbleibt, welche durch weiteres Vorschieben des IUP nach distal überwunden werden müßte. Beim weiteren Vorschieben besteht jedoch wieder das Problem, daß der Gleitwiderstand der Vorrichtung im Gebärmutterhalskanal überwunden werden muß, und somit ein Ertasten der cranialen Wand der Gebärmutterhöhle sehr schwierig ist. Weiterhin muß vor dem Entfernen des Einführungsgerätes der Stempel entfernt werden, da die Fäden des IUP sich zwischen Stempel und Hohlstab verklemmen könnten. Alle oben genannten Nachteile werden bei dem im Folgenden beschriebenen erfindungsgemäßen Einführungsgerät eliminiert.

In der Auslegeschrift DE 2513241 wird ein Insertionsgerät beschrieben, bei dem ein IUP mit nach proximal zum Spiralenkörper gebogenen Armen in einer Kappe zu liegen kommt. Diese Kappe wird mit einem distalen Flansch bis zum äußeren Gebärmutterhals geführt. Dann wird mit einem Rohr das IUP mit den nach proximal geklappten Armen durch den Gebärmutterhals geschoben. Die Kappe hat zwei U-förmige Aussparungen, welche als Sichtfenster dienen, damit der Arzt das nach distal gleitende IUP beobachten kann. Nachteilig bei dieser Ausführung ist, daß das IUP mit nach proximal geklappten Armen einen wesentlich größeren Querdurchmesser besitzt als zum Beispiel mit nach distal geklappten Armen, wodurch die Einlage traumatischer und schmerzhafter wird und es somit leicht zu Verletzungen des Gebärmutterhalskanales kommen kann. Die Kappe befindet sich außerhalb der Gebärmutter, es ist somit keine Lateralausrichtung des IUP in der Gebärmutterhöhle gewährleistet. Zwei Schlitze in der Kappe dienen dazu das IUP vor dem Einlegevorgang zu positionieren, indem die Arme nach proximal fixiert werden. Die Ausrichtung des IUP kann sich jedoch beim Vorschieben durch den Gebärmutterhalskanal leicht ändern. Im Gegensatz dazu werden beim erfindungsgemäßen nachfolgend beschriebenen Einlegegerät die Arme des IUP nach distal zusammengeklappt in einem Einführungsrohr gelagert, welches in die Gebärmutterhöhle geschoben wird und die Ausrichtung des IUP in der Gebärmutterhöhle in Lateral- und Horizontalrichtung definiert.

Der Erfindung liegt die Aufgabe zugrunde, ein Einführungsgerät für ein IUP zu schaffen, das die Einführung des Gerätes erleichtert und Verletzungen des Uterus beim Ausstoßen der empfängnisverhütenden Vorrichtung ausschließt.
Die Erfindung bezieht sich auf die Einlage von T-förmigen Spiralen wie z.B. die von der Firma Schering (Berlin) produzierten Typen Mirena (geschütztes Warenzeichen) und Nova-T (geschütztes Warenzeichen). Bei Spiralen dieser Art wird bisher die Spirale in einem Hohlstab mit nach oben geklappten Spiralenarmen gelagert. Die Spitzen der Spiralenarme liegen direkt hinter der distalen Öffnung des Hohlstabes. Die Fäden der Spirale werden durch den Hohlstab geführt und ragen aus der proximalen Öffnung des Hohlstabes heraus. Weiterhin ist ein Stempel von der proximalen Öffnung des Hohlstabes her in diesen bis zum Spiralenkörper hin eingeführt und dient dem Herausschieben der Spirale aus dem Hohlstab. Die Einlage der Spirale ist nun wie folgt, wobei bei diesem Einlagegerät ein zweihändiges Vorgehen notwendig ist. Zunächst wird der Gebärmutterhals mit einer Faßzange angehakt und durch Zug nach distal eine Streckung der Gebärmutter herbeigeführt. Dann wird der Hohlstab mit der Spirale nach Bestimmung der Sondenlänge und Einstellung derselben mit einem Schieber durch den Gebärmutterhalskanal geschoben bis der Schieber die Oberfläche des Gebärmutterhalses erreicht hat und dann das distale Ende des Hohlstabes an die craniale Begrenzung der Gebärmutterhöhle anstößt. Durch Zurückschieben des Hohlstabes gegen den Stempel wird die Spirale aus dem Hohlstab teilweise freigegeben, wobei die Lage des Hohlstabes zur Gebärmutter, zum Stempel und zur Faßzange dadurch verändert wird und in einem zweiten Schritt der Hohlstab zusammen mit dem Stempel unverschieblich zueinander festgehalten und nach distal vorgeschoben werden müssen um die korrekte Lage des IUP zu erreichen. Dann muß der Hohlstab bis zum Anschlag am Stempel nach proximal zurückgeschoben werden, um das IUP vollständig aus dem Hohlstab freizugeben, wobei hier wieder die Lage des Hohlstabes zur Gebärmutter, zum Stempel und zur Faßzange verändert wird. Das IUP muß aus dem Hohlstab geschoben werden, da sich dieser am distalen Ende im Durchmesser verjüngt, um die Einlage zu Erleichtern, jedoch dadurch den Spiralenkörper durch Gleitreibung am problemlosen Herausgleiten hindert. Dann muß der Hohlstab in seiner Lage unverändert bleiben und der Stempel vollständig entfernt werden, weil der Faden des IUP zwischen Stempel und Hohlstab eingeklemmt ist. Würde man den Stempel im Hohlstab belassen, und den Hohlstab zurückziehen, würde der Faden und somit das IUP mit zurückgezogen werden. Somit ergibt sich eine recht umständliche Handhabung, zumal zusätzlich noch in einer Hand die Faßzange gehalten werden muß, mit der der Gebärmutterhals zum Untersucher hin gezogen wird, um die Gebärmutter zu strecken.

Die nachfolgend beschriebene Erfindung bezieht sich auf ein spezielles Einführungsgerät, welches mit nur einer Hand zu bedienen ist und eine sichere und korrekte Plazierung des IUP in der Gebärmutterhöhle erlaubt. Die Handhabung ist klar definiert und einfach, das Verletzungsrisiko ist minimiert. Die Herstellung dieser Einführungsvorrichtung ist durch Modifikation des bekannten Einlegeinstrumentariums ohne großen zusätzlichen Herstellungsaufwand zu erreichen. Das Einführungsgerät mit integriertem IUP wird vom Hersteller des IUP üblicherweise als steril verpackte Kombination angeboten.

### Problemlösung:

Das erfindungsgemäße Einführungsgerät besteht aus einem Hohlstab, welcher am distalen Ende , in dem das IUP liegt mindestens 2 nach distal offene Längsschlitze besitzt, durch welche die Arme der Spirale ohne Reibung problemlos austreten können, sowie einen Stempel, welcher rohrförmig ausgebildet ist und welcher in den die Spirale aufnehmenden Hohlstab eingeführt werden kann und zum Herausschieben der Spirale dient. Durch das Stempelrohr laufen die Fäden der Spirale. Die Schlitze am distalen Ende des Hohlstabes liegen sich in einem Winkel von 180 Grad gegenüber und sind um einen definierten Abstand zwischen den noch nicht entfalteten Spiralenarmen und der Gebärmutterwand beim Einführen herzustellen entsprechend dimensioniert. Dieser Abstand ist so bemessen, daß beim Vorschieben des IUP die Arme sich entfalten können, ohne dabei die craniale Begrenzung der Gebärmutterhöhle zu berühren. Die Entfaltung der Spiralenarme wird beim Vorschieben mit dem Stempel erreicht, da diese durch die beiden Schlitze des Hohlstabes nach lateral federn. Um die Lage der Spirale im Hohlstab klar zu definieren, sind proximal der beiden Schlitze im Hohlstab Positionselemente angebracht, in welche die Verdickungen der Spiralenarme einrasten können. Die Anordnung der Positionselemente, welche im 180 Grad-Winkel angebracht sind, ist so gewählt, daß die Arme des IUP beim Vorschieben problemlos in die Schlitze des Hohlstabes einfedern können. Die Positionselemente können z.B. lokale Veränderung des Wanddurchmessers des Hohlstabes zur Lagedefinition des IUP darstellen und sind in den Abbildungen als Öffnung der Wand des Hohlstabes dargestellt. Die Positionselemente können auch Stellen mit größerem Innendurchmesser und somit geringer Wandstärke oder Stellen mit geringerem Innendurchmesser durch in das Lumen ragende Wandstrukturelemente oder bewegliche Wandteile oder Stellen mit verändertem Innen- und Außendurchmesser sein. Die Positionselemente dienen dazu das IUP im Hohlstab reversibel zu fixieren. Somit ist eine klare Lagedefinition geschaffen, wobei eine Markierung auf dem Hohlstab im Querschnitt bei 12 Uhr die Lage der Schlitze und der Arme des IUP bei exakt 9 und 3 Uhr anzeigt. Ein Schieber, welcher gegenüber dem Hohlstab schwer verschiebbar ist und diesen ganz oder teilweise zirkulär umgreift dient dazu die Sondenlänge, d.h. die mit der Uterussonde bestimmte Länge zwischen äußerem Muttermund des Gebärmutterhalses und der cranialen Begrenzung in der Gebärmutterhöhle einzustellen. Somit kann der Hohlstab nur bis maximal zum Erreichen der cranialen Begrenzung der Gebärmutterhöhle eingeführt werden und Verletzungen durch Perforationen durch den Hohlstab sind somit ausgeschlossen. Weiterhin ist ein Halteelement, welches mit dem Hohlstab verbunden ist an dessen proximalen Teil so angebracht daß ein ergonomisches Halten des Hohlstabes zwischen Zeige- und Mittelfinger möglich ist.

So wird also zunächst das IUP durch Einführen des Hohlstabes bis zum Anschlag des Schiebers am Gebärmutterhals in die Gebärmutter eingeführt. Die distalen Enden des Hohlstabes berühren mit ihren wulstigen Verdickungen die craniale Gebärmutterwand. Die distalen Kanten des Hohlstabes sind abgerundet um eine Gewebetraumatisierung zu verhindern. Die distalen Enden des Hohlstabes sind verdickt um einen höheren Gewebewiderstand wie bei dem eines kreisförmigen distalen Endes eines konventionellen Hohlstabes bzw. der Arme der Spirale in zusammengeklappten Zustand zu erreichen. Dieser Gewebewiderstand liegt somit höher als bei konventionellen Einlegevorrichtungen und bietet daher mehr Sicherheit. Die Materialbeschaffenheit soll so sein, daß die distalen Enden elastisch hin- und herfedern, also beim Einführen durch den Gebärmutterhals zum Kreiszentrum des Querschnittes des Hohlstabes federn können, und nach dem Erreichen der Gebärmutterhöhle in die Ausgangslage zurückfedern und den ursprünglichen Innenquerschnitt des Hohlstabes freigeben um diesen ohne Widerstand über den Spiralenkörper am Ende des Einführungsvorganges zurückschieben zu können. Dann wird im nächsten Schritt der Stempel mit dem Daumen an einem Vorschiebegriff nach distal geschoben, während der Hohlstab zwischen Zeige- und Mittelfinger am Halteelement gehalten wird. Der Stempel verschiebt sich leicht gegenüber dem Hohlstab, welcher durch einen Schieber auf die korrekte Sondenlänge eingestellt ist, und somit nicht weiter nach distal vorgeschoben werden kann. Der Vorschiebegriff des Stempels kann dazu aus einem Kunststoffteil bestehen, welches ergonomisch günstig geformt ist und eine zumindest weitgehend zirkuläre Öffnung besitzt, welche so weit ist, daß der Vorschiebegriff auf dem Stempel nur schwer hin- und hergeschoben werden kann. Dieser Vorschiebegriff ist so auf dem Stempel positioniert, daß dieser als Begrenzung beim Vorschieben des Stempels nach distal dient. So gleiten die Arme des IUP beim Vorschieben aus den Positionselementen heraus und federn nach lateral in die Längsschlitze hinein und entfalten sich. Der Stempel kann nun soweit vorgeschoben werden, bis die entfalteten horizontal liegenden Spiralenarme auf der Ebene des distalen Ende des Hohlstabes zu liegen kommen. In dieser Position wird das Einlegesystem entfernt, indem das Halteelement und der Vorschiebegriff in gleicher Position gehalten werden und gleichzeitig das gesamte Einführungsgerät nach proximal zurückgezogen wird und damit entfernt wird. Der Spiralenkörper gleitet dabei aus dem Hohlstab heraus, da die Spirale durch die entfalteten Arme in der Gebärmutterhöhle gehalten wird. Am Ende des Vorganges gleitet der Faden aus dem proximalen Ende des Stempel heraus. Alternativ kann das System so angewendet werden, daß nach dem Vorschieben des Vorschiebegriffes der Daumen gegen das proximale Ende des Stempels drückt und somit der Vorschiebegriff an das proximale Ende des Hohlstabes gedrückt wird. Der fühlbare Widerstand stellt den Druck der Arme des IUP gegen die craniale Wand der Gebärmutter dar. Dann kann das Halteelement und damit der Hohlstab nach proximal verschoben werden wobei der Stempel in seiner ursprünglichen Lage mit dem Daumen gehalten wird. Der Vorschiebegriff auf dem Stempel wird dabei durch den Hohlstab soweit über den Stempel zurückgeschoben, bis dieser eine Markierung auf dem Stempel erreicht. Der Schieber wird dadurch vom Gebärmutterhals distanziert. Wenn diese Markierung erreicht ist, zeigt dies an, daß der Hohlstab komplett hinter den Spiralenkörper zurückgeschoben wurde, und dieser jetzt frei in der Gebärmutterhöhle liegt. Jetzt wird der Daumen wieder vom Ende des Stempels entfernt und das Einführungsgerät wird nur durch Zug am Halteelement, welches zwischen Mittel- und Zeigefinger liegt nach proximal bewegt und damit entfernt. Der Vorgang ist beendet wenn die Fäden aus dem Stempel herausgegleitet sind. Der Faden kann durch den Hohlraum des Stempels ohne Widerstand hindurchgleiten.

Erläuterung der verwendeten medizinischen Begriffe:
Cranial heißt in Richtung zum Kopf hin der Patientin. Das caudale Ende weist in die entgegengesetzte Richtung.
In Bezug auf das Einführungsgerät bedeutet proximal den Teil des Gerätes, welcher zum Untersucher zeigt, distal den Teil der zur Patientin zeigt.
Die Einstellung des Schiebers auf dem Hohlstab ist mit schwer verschiebbar bezeichnet, was bedeutet, daß die Kräfte, welche notwendig sind diesen Schieber auf dem Hohlstab zu verschieben größer sein müssen als die üblicherweise aufgewendete Kraft, welche auf den Schieber wirkt, wenn dieser beim Einführen den Gebärmutterhals berührt. Der Schieber wird somit nicht in seiner Position verändert und zeigt dem Arzt somit an, daß die korrekte Sondenlänge erreicht ist. Der Vorschiebegriff kann ebenfalls nur schwer auf dem Stempel verschoben werden, somit ist beim Vorschieben des Stempels im Hohlstab der Widerstand zwischen Stempel und Hohlstab geringer als der Widerstand welcher zum Verschieben des Vorschiebegriffes notwendig wäre. Beim Einlegemodus, bei dem der Vorschiebegriff gegen den Stempel durch Zurückschieben des Hohlstabes nach proximal verschoben wird, geschieht dies gegen einen gewissen Widerstand, welcher zur kontrollierten Handhabung des Einlegegerätes beiträgt. Das Haltelement ist auf dem Hohlstab fixiert. Günstig ist dabei, daß alle Manipulationen durch die Finger einer Hand kontrolliert werden.

Eine bevorzuge Ausführungsform ist in den folgenden Abbildungen dargestellt.
Figur 1 zeigt einen Hohlstab, einen Stempel und eine empfängnisverhütende Vorrichtung (IUP) in Aufsicht.
Figur 2 zeigt das distale Ende des Einführungsgerätes in dreidimensionaler Darstellung.
Figuren 3 und 4 zeigen Schnittbilder durch Figur 2 im Lateral- und Frontalschnitt.
Figur 5 zeigt die Ansicht von Figur 4 in Aufsicht von distal.
Figur 6 zeigt das distale Ende des Einführungsgerätes beim Einführen durch den Gebärmutterhals.
Figuren 7-11 zeigen die Handhabung des Einführungsgerätes.

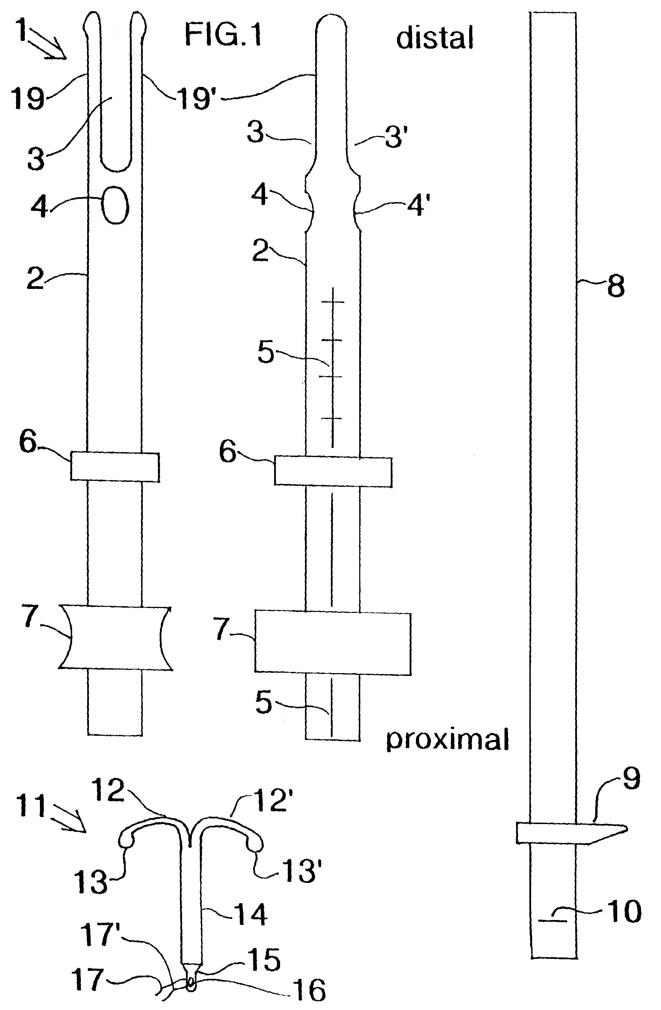

In Figur 1 ist ein Einführungsgerät 1 und eine empfängnisverhütende Vorrichtung 11 zu sehen. Das Einführungsgerät 1 besteht aus einem Hohlstab 2 und einem Stempel 8. Der Hohlstab 2 ist in Lateral- und Frontalansicht dargestellt. Die distalen und proximalen Enden sind ebenfalls bezeichnet. Der Hohlstab 2 besitzt am distalen Ende mindestens zwei nach distal offene Längsschlitze 3 und 3', welche nach lateral ausgerichtet sind und sich in einem Winkel von 180 Grad gegenüberliegen. Proximal der Längsschlitze 3 und 3' sind zwei Positionierungselemente 4 und 4' auf zwei Längsachsen, welche durch die Mitte der Längsschlitze 3 und 3' laufen mittig angeordnet. In diesen Positionierungselemente 4 und 4' kommen die Verdickungen 13 und 13' der Arme 12 und 12' zum liegen und definieren die Lage der empfängnisverhütenden Vorrichtung 11 im Hohlstab 2. Durch Schub des IUP nach distal gleiten die Verdickungen 13 und 13' aus den Positionierungselemente 4 und 4' und federn in die Längsschlitze 3 und 3' ein. Der Hohlstab 2 besitzt am proximalen Ende ein Halteelement 7, welches die Außenfläche des Hohlstabes zumindest teilweise zirkulär umfaßt und fest mit dem Hohlstab 2 verbunden ist und in einer vorteilhaften Ausführung eine ergonomische Form besitzt, so daß dieses zwischen Zeige- und Mittelfinger gehalten werden kann. Weiterhin ist ein Schieber 6 schwer verschiebbar am Hohlstab 2 angebracht, welcher die Außenfläche des Hohlstabes 2 zumindest teilweise zirkulär umfaßt und durch Gleitreibung auf dieser schwer verschiebbar ist. Auf der Frontalseite (im Querschnitt und Aufsicht von proximal bei 12 Uhr) des Hohlstabes 2 ist eine Markierung 5 zur Einstellung der Sondenlänge in Zentimetern vorhanden, um den Schieber 6 darauf einstellen zu können. Die Sondenlänge bedeutet die Länge in Zentimetern, welche vom distalen Ende des Hohlstabes 2 bis zur distalen Kante des Schiebers 6 gemessen wird. Die distale Kante berührt beim Einführen dann den Gebärmutterhals und das System wird dadurch am weiteren Vorschieben nach distal gehindert. Der Stempel 8 besitzt am proximalen Ende einen Vorschiebegriff 9, welcher die Außenfläche des Stempels 8 zumindest teilweise zirkulär umfaßt und auf dieser schwer verschiebbar ist. In einer vorteilhaften Ausführung ist der Vorschiebegriff 9 ergonomisch so geformt, daß der Daumen von proximal her in einer Mulde des Griffes zu liegen kommt, und dieser so den Griff bequem nach distal drücken kann. Am proximalen Ende des Stempels 8 ist eine zirkuläre Markierung 10 angebracht, welche die Lage zwischen Hohlstab 2 und Stempel 8 definiert. Wird der Hohlstab 2 gegen den Stempel 8 nach proximal verschoben bis die Markierung 10 vom proximalen Ende des Stempels 8 beziehungsweise dem Vorschiebegriff erreicht wird, kennzeichnet dies die Situation wenn der größte Umfang des Spiralenkörpers 14 distal des Hohlstabes 2 liegt und somit freigegeben ist. Der Vorschiebegriff wird dabei auf dem Stempel nach proximal geschoben. Die empfängnisverhütende Vorrichtung 11 besteht aus einem zentralen zylinderförmigen Spiralenkörper 14 an dessen cranialem Ende zwei elastische Arme 12 und 12' befestigt sind. Die lateralen Enden der Arme 12 und 12' besitzen je eine Verdickung 13 und 13'. Am caudalen Ende des Spiralenkörpers 14 ist ein Befestigungsteil 15 angeordnet, das in seiner Mitte ein Auge 16 besitzt. Durch das ein oder zwei Kunststoffäden 17 und 17' gezogen sind.

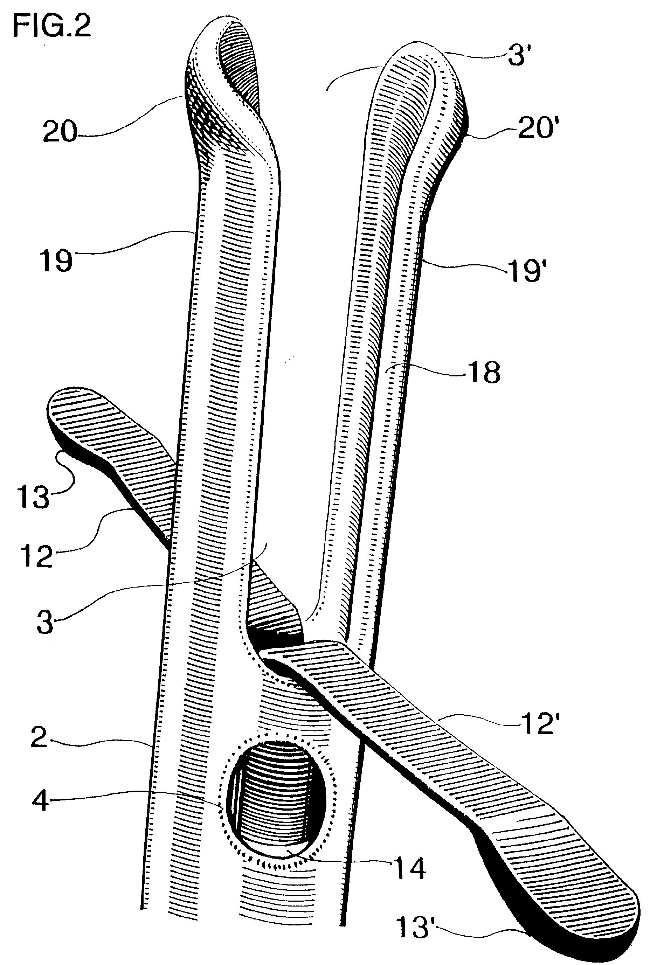

Figur 2 zeigt das distale Ende des Hohlstabes 2. In der dreidimensionalen Abbildung erkennt man die sich gegenüberliegende Längsschlitze 3 und 3' sowie ein proximal davon angeordnetes Positionierungselement 4. Die Endkante 18 des Hohlstabes 2 ist im Querschnitt abgerundet und verläuft kurvenförmig. Die distalen Enden 19 und 19' des Hohlstabes 2 besitzen wulstförmige Verdickungen 20 und 20*'*, welche den Innendurchmesser des Hohlstabes nicht einengen sondern den Außendurchmesser vergrößern.

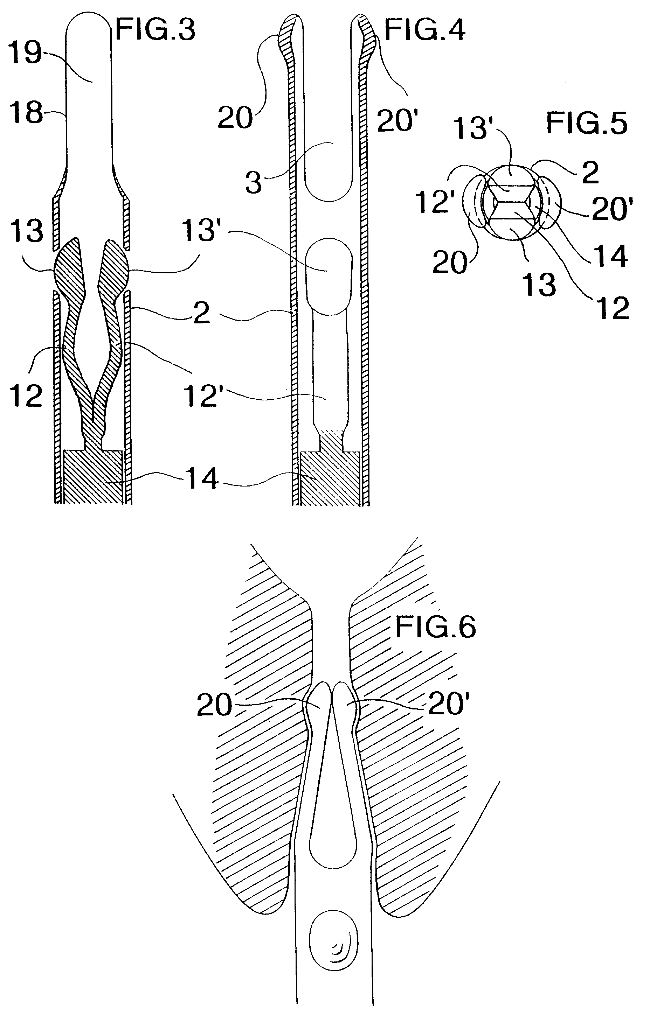

Figur 3 zeigt einen Längsschnitt (frontal) durch das distale Ende des Hohlstabes 2 mit eingelegter empfängnisverhütender Vorrichtung 11. Die Verdickungen 13 und 13' der Arme 12 und 12' liegen teilweise in den Positionierungselemente 4 und 4'.

Figur 4 zeigt einen Längsschnitt (lateral) durch das distale Ende des Hohlstabes 2 mit eingelegter empfängnisverhütender Vorrichtung 11.

Figur 5 zeigt die Aufsicht auf Figur 4 von distal.

Figur 6 zeigt das distale Ende des Einführungsgerätes in Situ beim Einführen durch den Gebärmutterhalskanal. Die distalen Enden 19 und 19' können beim Einführen durch den Gebärmutterhals durch Verwendung eines Materials mit Gedächtniseffekt zum Zentrum des Innendurchmessers des Hohlstabes 2 zusammenfedern und durch Berührung ihrer verdickten Enden eine kugelförmige Spitze bilden, da im Gebärmutterhals das umliegende Gewebe einen entsprechenden Druck zum Zentrum hin ausübt. Somit vermindert sich dadurch der Außendurchmesser des distalen Endes des Hohlstabes 2, was sich beim Einführen des Hohlstabes 2 durch den Gebärmutterhalskanal sehr positiv bemerkbar macht, da dieser so beim Vorschieben des Hohlstabes zunächst von der distalen Spitze mit geringerem Durchmesser passiert wird und dann beim weiteren Vorschieben zum endgültigen Außendurchmesser des Hohlstabes 2 stufenlos aufgedehnt wird. Damit ist auch die Einlage eines Gestagen- IUP z.B. vom Typ Mirena (geschütztes Warenzeichen, Firma Schering), welches im Vergleich zu den konventionellen IUP einen größeren Durchmesser des Spiralenkörpers 14 besitzt, erleichtert. Beim konventionellen Einlegesystem besitzt das distale Ende nahezu den gesamten Außendurchmesser des Hohlstabes 2. Dieser muß durch den Gebärmutterhalskanal geschoben werden, wobei durch den großen Durchmesser eine plötzliche Dehnung des Gewebes verursacht wird und daher Schmerzen auftreten können. Manchmal gelingt es nicht den Gebärmutterhalskanal mit dem Hohlstab zu passieren, so daß eine vorhergehende Dilatation mit Hegarstiften nötig sein kann, welche nicht ohne Anästhesie durchgeführt werden kann und somit den Eingriff für die Patientin erweitert. Erreicht das distale Ende des Hohlstabes 2 die weite Gebärmutterhöhle, federn die Enden 19 und 19' wieder in Ausgangsposition zurück und geben damit den ursprünglichen Innendurchmesser des Hohlstabes 2 wieder frei.

Die distale Kante 18 des Hohlstabes 2 soll im Querschnitt gerundet sein um die Gewebetraumatisierung beim Einlegen so gering wie möglich zu halten und im Verlauf keine scharfen Kanten oder Ecken aufweisen, also kurvenartig verlaufen. Die Länge der Längsschlitze 3 und 3' ist so bemessen, daß zwischen distalen Ende 19 und 19' des Hohlstabes 2 und den proximalen Begrenzungen der Längsschlitze 3 und 3' so viel Raum bleibt, daß die Arme 12 und 12' sich beim Vorschieben nach lateral entfalten können, ohne die Ebene des distalen Endes des Hohlstabes 19 und 19', welche der Gebärmutterschleimhaut anliegt zu berühren. Nur so kann eine korrekte Entfaltung bei definierter Lage in der Gebärmutterhöhle gewährleistet werden. Die Breite der Längsschlitze 3 und 3' ist so gewählt, daß die Arme 12 und 12' ohne Reibung in die Schlitze einfedern können und sich so lateral entfalten können. Die Positionieruingselemente 4 und 4' sind so angeordnet, daß die Verdickungen das IUP 13 und 13' teilweise in Öffnungen der Wand einfedern ohne die Außenfläche des Hohlstabes 2 zu überragen. Somit ist die Lage der Spirale im Hohlstab klar definiert. Bei Schub der Vorrichtung 11 nach distal gleiten die Arme 12 und 12' leicht aus der Öffnung der Positionierungselemente in Richtung auf die Längsschlitze 3 und 3' heraus und federn in diese Längsschlitze ein und entfalten sich dabei nach lateral. Die Begrenzungen der als Fenster dargestellten Positionierungselemente sind im Querschnitt abgerundet, die Form der Positionierungselemente ist den Verdickungen 13 und 13' angepaßt. Die Positionierungselemente sollen die Arme 12 und 12' bis zum Einfedern in die Längsschnitte 3 und 3' führen

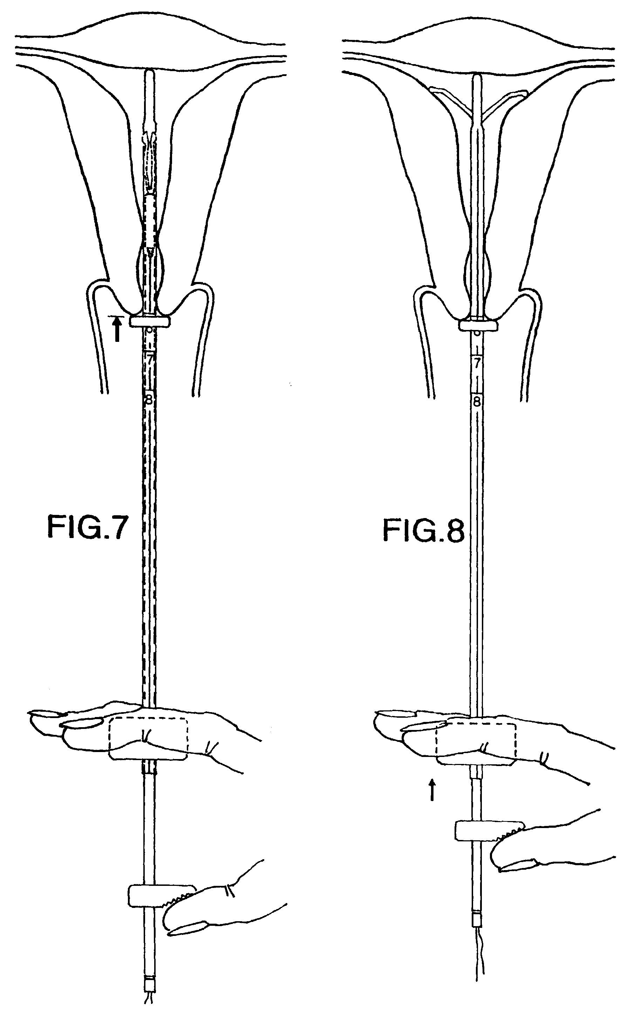

Figur 7 zeigt das Einführungsgerät, welches soweit eingeführt wurde, bis der Schieber den Gebärmutterhals berührt.

Figur 8 zeigt, daß bei Betätigung des Vorschiebegriffes die Spiralenarme sich nach lateral entfalten, jedoch noch eine Distanz zur cranialen Gebärmutterwand vorhanden ist.

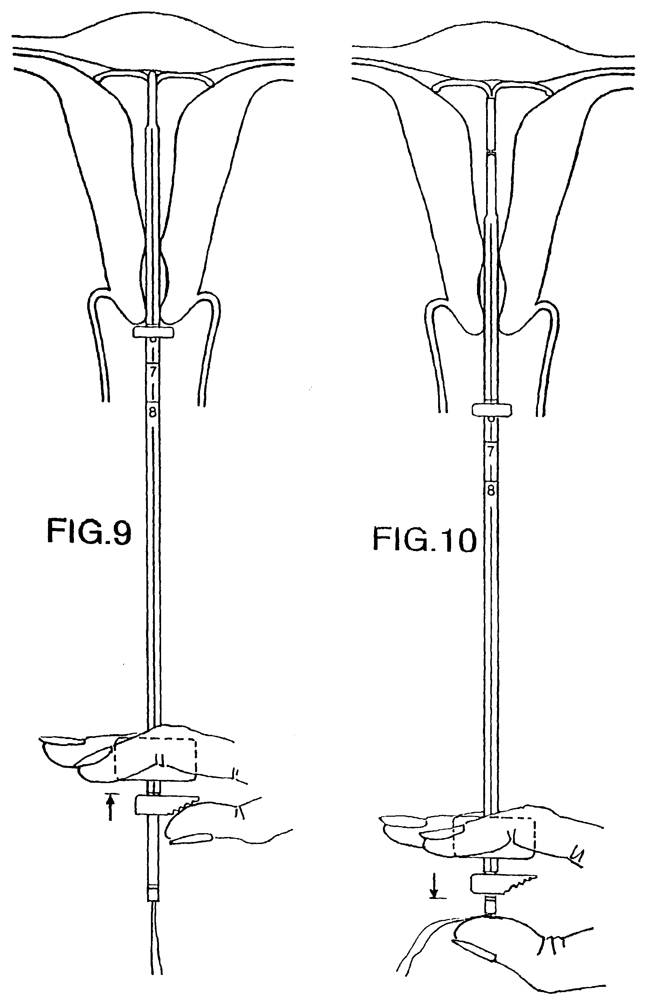

Figur 9 zeigt, daß bei vollständig vorgeschobenem Vorschiebegriff die Spiralenarme durch den Stempel soweit vorgedrückt werden, bis diese ihre endgültige Position erreicht haben und der cranialen Wand der Gebärmutterhöhle anliegen.

Figur 10 zeigt, wie der Stempel mit dem Daumen vom proximalen Ende her gehalten wird. Ist der Hohlstab gegen den Stempel bis zur Markierung zurückgeschoben, liegt der Spiralenkörper frei in der Gebärmutterhöhle und wird nicht mehr vom Hohlstab umschlossen.

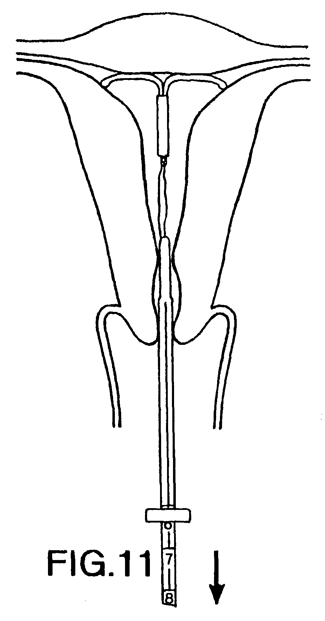

Figur 11 zeigt das Einlegegerät während der Entfernung aus der Gebärmutterhöhle.

Der in Figur 10 dargestellte Schritt kann entfallen, wenn das IUP leicht aus dem Hohlstab herausgleiten kann, d.h. wenn der Gleitwiderstand des Spiralenkörpers im Hohlstab geringer ist, als die Kraft, welche von den Spiralenarmen ausgeht und das IUP am Herausgleiten aus der Gebärmutterhöhle hindern will. Somit kann bei entsprechender Konstruktion des Hohlstabes der in Figur 10 dargestellte Schritt entfallen, und nach dem in Figur 9 dargestelltem Schritt direkt das Entfernen des Einführungsgerätes wie in Figur 11 dargestellt erfolgen.

### Bezeichnungsliste:

- 1: = Einführungsgerät
- 2: = Hohlstab
- 3, 3': = Längsschlitze
- 4, 4': = Positionierungselemente
- 5: = Markierung
- 6: = Schieber
- 7: = Halteelement
- 8: = Stempel
- 9: = Vorschiebegriff
- 10: = Markierung
- 11: = empfängnisverhütende Vorrichtung
- 12, 12': = Arme
- 13, 13': = Verdickungen
- 14: = Spiralenkörper
- 15: = Befestigungsteil
- 16: = Auge
- 17, 17': = Kunststoffäden
- 18: = abgerundete Endkante des Hohlstabes 2
- 19: = distales Ende des Hohlstabes 2
- 20, 20': = wulstige Verdickungen

## Patentansprüche

1. Einführungsgerät für die Einlage einer intrauterinen, empfängnisverhütenden Vorrichtung(11), bestehend aus einem Hohlstab(2) zur Aufnahme der empfängnisverhütenden Vorrichtung(11), mit
a)einem Positionierungselement(4,4*'*) für jeden Arm(12,12*'*) der empfängnisverhütenden Vorrichtung(11) zu deren definierten Lagerung im Hohlstab(2) und
b)pro Arm(12,12*'*) der empfängnisverhütenden Vorrichtung(11) mit einem parallel zur Längsachse des Hohlstabes(2) verlaufenden und zum distalen Ende hin offenen Längsschlitz(3,3*'*) mit einer an den Armdurchmesser angepaßten Breite in der am distalen Ende verdickten Wand des Hohlstabes(2), sowie
c)einem auf der Mantelfläche des Hohlstabes(2) verschiebbaren Schieber(6) zur Einstellung der Länge eines Abschnitts des Hohlstabes, der beim Gebrauch in den Uterus einer Benutzerin der empfängnisverhütenden Vorrichtung(11) einführbar ist,
d)einem im Hohlstab(2) verschiebbaren, rohrförmigen Stempel(8), auf dessen Mantelfläche ein Vorschiebegriff(9) verschiebbar angeordnet ist, dessen Haftreibung auf der Mantelfläche des Stempels(8) größer ist als die gemeinsame Haft- und die gemeinsame Gleitreibung des Stempels(8) und der empfängnisverhütenden Vorrichtung (11) im Hohlstab(2).

2. Einführungsgerät nach Anspruch 1 dadurch gekennzeichnet, daß Kohlstab(2) und Stempel(8) aus Kunststoff gefertigt sind und flexibel sind.

3. Einführungsgerät nach Anspruch 1 oder 2 dadurch gekennzeichnet, daß die Positionierungselemente(4,4*'*) in Form von flächigen Vergrößerungen des Innendurchmessers oder flächige Verkleinerungen des Innendurchmessers durch in das Lumen vorspringende Strinkturelemente der Wand oder federnde Elemente der Wand oder Stellen mit vergrößertem Innen- und Außendurchmesser oder Öffnungen der Wand sind, in welche Verdickungen der Arme(12,12*'*) reversibel einfedern und proximal der Längsschlitze angeordnet sind.

4. Einführungsgerät nach einem der Ansprüche 1-3 dadurch gekennzeichnet, das am proximalen Ende des Hohlstabes(2) auf dessen Mantelfläche ein Halteelement(7) fest angebracht ist.

5. Einführungsgerät nach einem der Ansprüche 1-4 dadurch gekennzeichnet, daß eine Markierung(5) zur Einstellung des Schiebers(6) auf dem Hohlstab(2) vorgesehen ist.

6. Einführungsgerät nach einem der Ansprüche 1-5 mit einem Hohlstab dadurch gekennzeichnet, daß dieser eine Markierung zur lateralen Ausrichtung der Arme der empfängnisverhütenden Vorrichtung hat.

7. Einführungsgerät nach einem der Ansprüche 1-6 dadurch gekennzeichnet, daß die Kanten der Längsschlitze(3,3*'*) im Querschnitt abgerundet sind und der Verlauf der distalen Endkante kurvenförmig ist.

8. Einführungsgerät nach Anspruch 3 dadurch gekennzeichnet, daß die Begrenzungen der Positionierungselemente im Querschnitt abgerundet sind.

9. Einführungsgerät nach einem der Ansprüche 1-8 dadurch gekennzeichnet, daß die distalen Enden wulstförmig verdickt sind ohne dabei den Innendurchmesser des Hohlstabes zu verkleinern.

10. Einführungsgerät nach einem der Ansprüche 1-9 dadurch gekennzeichnet, daß die distalen Enden durch Druck von lateral gegen das Kreiszentrum des Querschnittes des Hohlstabes gedrückt werden können und beim Nachlassen des lateralen Druckes wieder in ihre Ausgangsposition zurückfedern.

11. Einführungsgerät nach einem der Ansprüche 1-10 dadurch gekennzeichnet, daß der Innendurchmesser des Stempels(8) kleiner oder gleich dem Außendurchmesser der empfängnisverhütenden Vorrichtung im zusammengefalteten Zustand ist.

12. Einführungsgerät nach einem der Ansprüche 1-11 dadurch gekennzeichnet, daß ein Befestigungsteil(15) der empfängnisverhütenden Vorrichtung ohne Reibung in das Stempelrohr einführbar ist.

13. Einführungsgerät nach Anspruch 12 dadurch gekennzeichnet, daß am Befestigungsteil(15) Fäden vorgesehen sind, deren Reibung an der Innenwand des Stempels(8) vernachlässigbar ist.

14. Einführungsgerät nach einem der Ansprüche 1-13 dadurch gekennzeichnet, daß der Stempel(8) eine Markierung(10) zur Kennzeichnung der Lage des Hohlstabes(2) zum Stempel(8) besitzt.
